Europäisches Patentamt

European Patent Office

Office européen des brevets

(11) Veröffentlichungsnummer: **0 025 535**
A2

# (12) EUROPÄISCHE PATENTANMELDUNG

(21) Anmeldenummer: 80105137.6

(22) Anmeldetag: 28.08.80

(51) Int. Cl.³: **F 24 D 19/00**, F 24 F 6/00

(30) Priorität: 12.09.79 DE 2936895

(43) Veröffentlichungstag der Anmeldung: 25.03.81
Patentblatt 81/12

(84) Benannte Vertragsstaaten: AT BE CH FR GB IT LI LU NL SE

(71) Anmelder: SIEMENS AKTIENGESELLSCHAFT Berlin und München, Postfach 22 02 61, D-8000 München 22 (DE)

(72) Erfinder: Zoeke, Siegfried, Ing.grad., Balanstrasse 77, D-8000 München 80 (DE)
Erfinder: Stürzer, Anton, Ing.grad., Inntalstrasse 6, D-8018 Grafing (DE)

(54) Vorrichtung zur Steuerung der Verdampfung einer Flüssigkeit.

(57) Eine Flüssigkeit wird durch eine Verdampfereinrichtung verdampft, die aus einem Behälter (10) und einem Heizelement (12) besteht. Zur Steuerung der Verdampfung durch Ein- und Ausschalten des Heizelementes ist der Behälter (10) auf einer Platte (16) angeordnet, die auf einer ihrer Seiten über ein Federblech (18) an einem Gestell (20) befestigt ist. Mit einer weiteren Seite betätigt die Platte (16) einen Mikroschalter (22), durch den das Heizelement ein- oder ausgeschaltet wird.

SIEMENS AKTIENGESELLSCHAFT     Unser Zeichen

BERLIN UND MÜNCHEN     VPA **79 P 2 0 5 7 EUR**

Vorrichtung zur Steuerung der Verdampfung einer Flüssigkeit

Die Erfindung bezieht sich auf eine Vorrichtung zur Steuerung der Verdampfung einer Flüssigkeit mittels einer aus einem die Flüssigkeit aufnehmenden Behälter und einem die Flüssigkeit erwärmenden Heizelement bestehenden Verdampfereinrichtung.

Bei einer Verdampfereinrichtung, bei der ständig zu verdampfende Flüssigkeit zugeführt wird, muß darauf geachtet werden, daß die Verdampfung der Flüssigkeit dann beginnt, wenn dem Behälter genügend Flüssigkeit zugeführt worden ist und die Verdampfung der Flüssigkeit beendet wird, wenn die im Behälter enthaltene Flüssigkeit verdampft ist. Es muß somit eine Steuerung vorhanden sein, durch die das die Flüssigkeit erwärmende Heizelement zu den richtigen Zeitpunkten ein- bzw. ausgeschaltet wird.

Il 1 Stl / 7.9.1979

Die der Erfindung zugrundeliegende Aufgabe besteht darin, eine Vorrichtung zur Steuerung der Verdampfung einer Flüssigkeit bei der oben angegebenen Verdampfereinrichtung anzugeben, die äußerst kostengünstig ist. Diese Aufgabe wird dadurch gelöst, daß der Behälter auf einer Platte angeordnet ist, daß die Platte auf der einen Seite über ein Federblech an einem Gestell befestigt ist und daß ein durch die Platte schaltbarer Mikroschalter vorgesehen ist, durch den das Heizelement ein- bzw. ausgeschaltet wird.

Zur Meldung von Störfällen ist es zweckmäßig, einen zweiten durch die Platte schaltbaren Mikroschalter vorzusehen, der bei Erreichen einer maximalen Füllung des Behälters eingeschaltet wird und damit angibt, daß nicht mehr genügend Flüssigkeit verdampft wird.

Zur weiteren Überwachung der Vorrichtung ist es zweckmäßig, in die elektrische Zuleitung zum Heizelement eine Temperatursicherung anzuordnen, die feststellt, ob das Heizelement zu stark erwärmt wird.

Besonders einfach ist der Aufbau der Vorrichtung, wenn die Platte als Heizelement ausgeführt ist.

Anhand eines Ausführungsbeispieles, das in den Figuren dargestellt ist, wird die Erfindung weiter erläutert. Es zeigen

FIG 1     eine Seitenansicht der Vorrichtung,

FIG 2     eine Draufsicht auf die Vorrichtung,

FIG 3     ein Prinzipschaltbild der Steuerung.

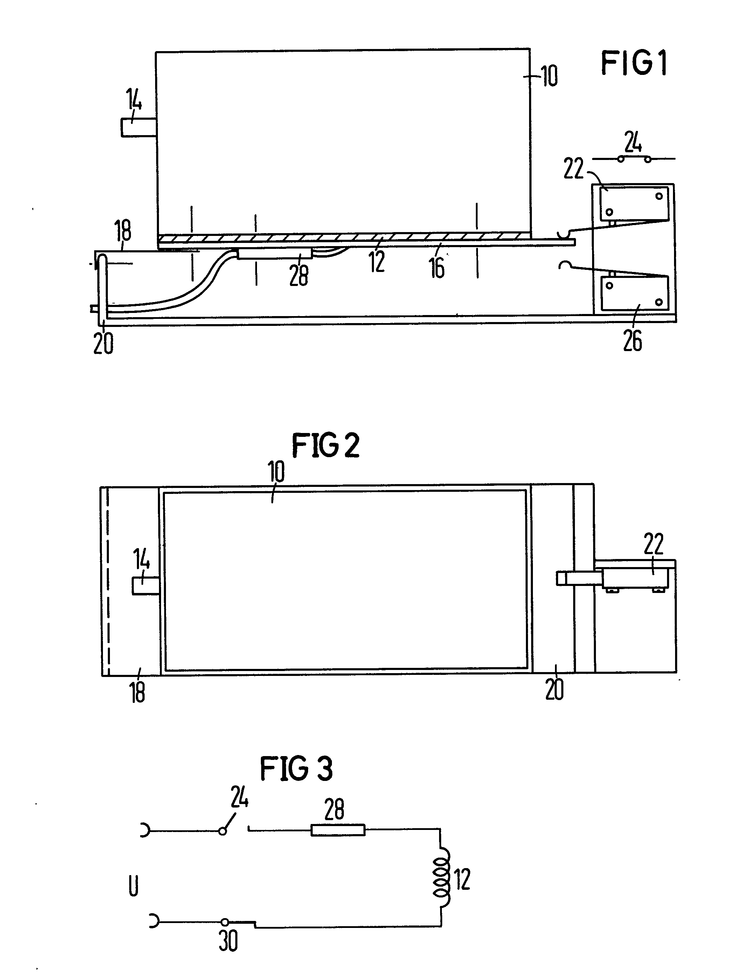

Aus FIG 1 und FIG 2 ergibt sich eine Verdampfereinrichtung, die aus einem Behälter 10 und einem Heizelement 12 besteht. Dem Behälter 10 kann über eine
Rohrleitung 14 Flüssigkeit,z.B. Wasser,zugeführt
werden. Zur Verdampfung der Flüssigkeit im Behälter 10
wird das Heizelement 12, z.B. eine Heizplatte, eingeschaltet und dadurch die Flüssigkeit solange erwärmt
bis diese verdampft. Dabei ist es zweckmäßig, die
Heizung des Behälters 10 sowie die Behältergröße
so zu dimensionieren, daß die maximal anfallende Flüssigkeitsmenge pro Zeiteinheit verdampft wird, ohne daß
der Behälter damit vollständig geleert wird.

Der Behälter 10 ist auf einer Platte 16 angeordnet.
Auf der einen Seite der Platte 16 ist ein Federblech 18
befestigt, das seinerseits an einem Gestell 20 angeordnet ist. Dabei ist der Behälter 10 ausschließlich
über das Federblech 18 am Gestell 20 befestigt.

An einer weiteren Seite der Platte 16, die z.B. dem
Federblech 18 gegenüberliegt, kann ein erster Mikroschalter 22 so angeordnet sein, daß er von der Platte
16 ein- und ausschaltbar ist. Hat dabei der Behälter 10
und damit die Platte 16 eine erste obere Stellung, bei
der der Behälter leer ist, dann ist der Mikroschalter
22 geöffnet. Wird Flüssigkeit in den Behälter 10 gefüllt, so senkt sich dieser über das Federblech 18
durch das Gewicht der Flüssigkeit ab und der Mikroschalter 22 wird eingeschaltet.

Der zum Mikroschalter 22 gehörende Kontakt 24 ist
in einer Steuerschaltung angeordnet, durch die das
Ein- und Ausschalten des Heizelementes 12 gesteuert
wird. Wenn der Behälter 10 leer ist, dann ist der Kontakt 24 geöffnet und es kann kein elektrischer Strom zum Heizelement 12 fließen. Wird jedoch aufgrund von Flüssigkeit der Behälter 10 abgesenkt, dann wird der Kontakt 24 geschlossen und damit die Stromzufuhr zum Heizelement 12 ermöglicht. Damit kann die Flüssigkeit erwärmt werden und verdampfen.

Die Überwachung der Vorrichtung kann mit Hilfe eines zweiten Mikroschalters 26 durchgeführt werden. Dieser ist unterhalb des ersten Mikroschalters 22 angeordnet und kann ebenfalls von der Platte 16 ein- oder ausgeschaltet werden. Im Normalfall ist dabei der Mikroschalter 26 eingeschaltet, d.h. die Platte 16 hat den Mikroschalter 26 nicht betätigt. Wenn nun die Flüssigkeitsmenge im Behälter 10 ansteigt, z.B. weil die ankommende Flüssigkeit von dem Heizelement 12 nicht mehr verdampft werden kann, dann sinkt der Behälter 10 ab. Bei Erreichen einer festlegbaren maximalen Flüssigkeitsmenge im Behälter betätigt die Platte 16 den Mikroschalter 26, d.h. ein dem Mikroschalter 26 zugeordneter Kontakt wird geöffnet. Damit wird ein Störfall angezeigt. Ein solcher Störfall kann z.B. auftreten, wenn dem Behälter 10 zuviel Flüssigkeit zugeführt wird, wenn das Heizelement 12 nicht eingeschaltet wird oder mangelhaft ist, z.B. weil der Mikroschalter 22 ausfällt.

Ein weiterer Störfall wird mit Hilfe einer Temperatursicherung 28 festgestellt. Wird nämlich zuviel Flüssigkeit verdampft, dann wird mit zunehmender Zeit der Behälter 10 entleert und das Heizelement 12 zu stark erhitzt. Dies stellt die Temperatursicherung 28 fest, die den Stromzufluß zum Heizelement 12 unterbricht. Ein solcher Störfall kann z.B. dadurch eintreten, weil der Mikroschalter 22 das Heizelement nicht mehr ausschaltet. Wird nun weiterhin Flüssigkeit in den Behälter 10 geliefert, dann sinkt der Behälter 10 aufgrund seines Gewichtes ab und betätigt den Mikroschalter 26, der den Störfall anzeigt.

Eine prinzipielle Steuerschaltung ergibt sich aus FIG 3. Hier ist in einem Stromkreis, der an seine Spannungsquelle U angeschlossen ist, der Kontakt 24 des Mikroschalters 22, die Temperatursicherung 28, das Heizelement 12, dargestellt als Heizspirale, und ein Kontakt 30 des Mikroschalters 26 angeordnet. Wenn der Behälter 10 leer ist, dann ist der Kontakt 24 geöffnet, während der Kontakt 30 geschlossen ist. Es kann kein Strom durch das Heizelement 12 fließen. Wird dem Behälter 10 Flüssigkeit zugeführt, dann wird der Kontakt 24 geschlossen, es kann Strom von der Spannungsquelle U über den Kontakt 24, die Temperatursicherung 28, das Heizelement 12 und den Kontakt 30 fliessen. Damit wird das Heizelement erwärmt. Wenn ein Störfall auftritt, wird der Kontakt 30 des Mikroschalters 26 geöffnet und damit wiederum ein Stromfluß durch das Heizelement 12 auf jeden Fall unterbunden. Gleichzeitig gibt die Stellung des Kontaktes 30 an, ob ein Störfall vorliegt oder nicht.

Im Ausführungsbeispiel der FIG 1 ist das Heizelement 12 im Boden des Behälters 10 angeordnet. Es ist aber auch möglich, als Heizelement eine Heizplatte zu verwenden, auf die der Behälter 10 gestellt wird. Dann kann diese Heizplatte als Platte 16 die Mikroschalter 22 und 26 betätigen.

4 Patentansprüche
3 Figuren

Patentansprüche

1. Vorrichtung zur Steuerung der Verdampfung einer Flüssigkeit mittels einer aus einem die Flüssigkeit aufnehmenden Behälter und einem die Flüssigkeit erwärmenden Heizelement bestehenden Verdampfereinrichtung, dadurch gekennzeichnet, daß der Behälter (10) auf einer Platte (16) angeordnet ist, daß die Platte (16) auf der einen Seite über ein Federblech (18) an einem Gestell (20) befestigt ist und daß ein durch die Platte schaltbarer Mikroschalter (22) vorgesehen ist, durch den das Heizelement (12) ein- oder ausschaltbar ist.

2. Vorrichtung nach Anspruch 1, dadurch gekennzeichnet, daß ein zweiter durch die Platte (16) schaltbarer Mikroschalter (26) vorgesehen ist, der bei Erreichen einer maximalen Füllung des Behälters (10) einschaltbar ist.

3. Vorrichtung nach Anspruch 1 oder 2, dadurch gekennzeichnet, daß in der elektrischen Zuleitung zum Heizelement (12) eine Temperatursicherung (28) in der Nähe des Heizelementes angeordnet ist.

4. Vorrichtung nach einem der vorhergehenden Ansprüche, dadurch gekennzeichnet, daß die Platte (16) als Heizelement ausgeführt ist.

FIG 1

FIG 2

FIG 3